# EUROPEAN PATENT APPLICATION

(11) **EP 3 656 378 A1**
(43) Date of publication of application: **27.05.2020**
(21) Application number: 18208392.3
(22) Date of filing: 26.11.2018
(51) Int. Cl.: A61K 9/107, A61K 31/167, A61P 17/02, A61P 15/00, A61P 17/04, A61P 27/00, A61P 23/02, A61P 17/10, A61P 17/00, A61K 9/00, A61K 47/34

(54) **NEW COMPOSITION OF AMIDE AND ESTER LOCAL ANESTHETICS AND USES THEREOF**

(71) Applicant: ValMeyer Sàrl, 1169 Yens (CH); Apidel SA, 1201 Geneva (CH)
(72) Inventor: MEYER, Marie, 1169 Yens (CH); GURNY, Robert, 1204 Geneva (CH)
(74) Representative: reuteler & cie SA

(57) **Abstract**

The present invention relates to novel topical compositions and uses thereof which are useful in tissue local anaesthesia, in particular in the context of the prevention and/or treatment of pain and/or a premature ejaculation. The invention further provides a process of preparation of novel topical compositions and topical articles for anaesthesia such as wound dressing or bandage, sponge, gauze or condom coated or impregnated with a composition of the invention.

## Description

### Field of the Invention

The present invention relates to a new composition of local anaesthetics and a process of preparation and uses thereof.

### Background of the Invention

Anaesthesia of the skin is needed to perform otherwise painful procedures such as perforation by needle for venous puncture in blood sampling and catheter placement, simple surgeries, skin debridement and laser treatments. The skin can also benefit from analgesia after minor traumas such as scrapes, insect bites or painful skin eruptions and rashes.

Currently, commonly used topical and local anaesthetic formulas are comprised mostly of high concentration of water-soluble lidocaine (4%) or a eutectic mixture of 2.5% lidocaine and 2.5% prilocaine (EMLA®, Allergan Inc.). Local anaesthetics such as lidocaine and others (e.g., bupivacaine, ropivacaine, revobupivacaine, chloroprocaine, tetracaine, etc.) act by blocking the sodium channels in the neurons, therefore inhibiting the electric transmission of information from one nerve cell to the other. Similar sodium channels are found in the brain and heart and therefore high concentrations of local anaesthetics entering the blood stream may provoke seizures or lethal cardiac rhythms. Therefore, topical or injected local anaesthetics have a maximal dose/quantity which can be administered without reaching the toxic blood levels responsible for the severe complications and as a drawback a limited area of the tissue may be anaesthetised at a given time. The EMLA® formulation is the most commonly used topical anaesthetic in Europe but has also the disadvantage of momentarily discolouring the skin rendering vessel puncture and small surgeries more difficult. In addition, prilocaine is known to cause a particularly dangerous health complication especially in small children called methemoglobinemia that is a change in the iron ionisation in haemoglobin that is increasing its affinity for oxygen and which lessens the oxygen release in tissues resulting in life-threatening hypoxia. Lidocaine is also known to cause methemoglobinemia, but to a much lesser extent. This condition is rare in adults but limits the application of EMLA® formulation on small infants and new-borns. Furthermore, EMLA®'s greasy formula has the disadvantage of needing a soap and water wash before bandage or adhesive dressing application after the treatment.

Block copolymers of methoxy poly(ethylene glycol) (mPEG) and poly(caprylic acid) have been developed for incorporation of various drugs during the spontaneous formation of nanosized micelles in a self-assembly process as described in WO 2012/014011 for a wide range of applications.

Therefore, in view of the possible side effects of excessive plasma concentrations of amide or ester local anaesthetics, there is a need to develop new formulations of those which would be efficient in providing the aimed anaesthetic effect but with a reduced risk of side effects.

### Summary of the Invention

The present invention is directed to the finding that a block copolymer of methoxy poly(ethylene glycol) (mPEG) and poly(caprylic acid) can efficiently solubilize amide or ester local anesthetics such as lidocaine into a biodegradable micellar formulation which allows the use of lower concentration of lidocaine (1.5%) with a similar anaesthetic action and onset time as compared to the EMLA® formulation comprising a mixture of 2.5% lidocaine and 2.5% prilocaine. The micellar formulation can be advantageously used for anaesthetising larger areas of tissue, in particular of the skin and as it avoids the use of prilocaine, it has less of the associated drawbacks (Suzuki et al., 2013, Pharmacology and Physiology for Anesthesia, Chap 17, 291-308*;* Black, 2015, Side Effects of Drugs Annual, 37, Chap 14, 175-184*).* Further, as opposed to the current state of the art topical anaesthetics formulation wherein the dissolved anaesthetics diffuse through the tissue barrier into the tissue and achieve their effect as soon as the target receptor is encountered, it has been found that a topical pharmaceutical composition of the invention, allows the amide or ester local anesthetics to pass the tissue barrier in a micellar formulation and those are dissolved only progressively in the physiological environment once in contact with the humidity of the live tissues of the epidermis, dermis and skin connective tissues after the disintegration of the micelles and thereby achieving a longer anaesthetic effect. This process advantageously prevents a quick exposure of the local anesthetics to the blood streams and therefore high toxic blood level peaks of those.

A first aspect of the invention provides a topical pharmaceutical composition comprising a block copolymer of methoxy poly(ethylene glycol) (mPEG) and poly(caprylic acid) and at least one amide or ester local anaesthetics.
Another aspect of the invention provides a topical pharmaceutical composition comprising a block copolymer of methoxy poly(ethylene glycol) (mPEG) and poly(caprylic acid) and at least one amide or ester local anaesthetics for local anaesthesia and treatment of topical irritations and premature ejaculation.

Another aspect of the invention provides a use of block copolymer of methoxy poly(ethylene glycol) (mPEG) and poly(caprylic acid) for the preparation of a topical pharmaceutical composition comprising at least one amide or ester local anesthetics for local anaesthesia and for the treatment of topical inflammation, abrasions, rashes, eruptions, cuts or irritations or premature ejaculation.
Another aspect of the invention provides a process for the preparation of a topical pharmaceutical composition of the invention.
Another aspect of the invention provides topical articles for anaesthesia such as wound dressing or bandage, sponge, gauze or condom coated or impregnated with a composition of the invention.
Another aspect of the invention provides a method for treating a subject suffering from a topical inflammation, abrasions, rashes, eruptions, cuts or irritations or premature ejaculation, wherein said method comprises topically treating the said subject with an effective amount of a topical composition of the invention.

### Brief description of the drawings

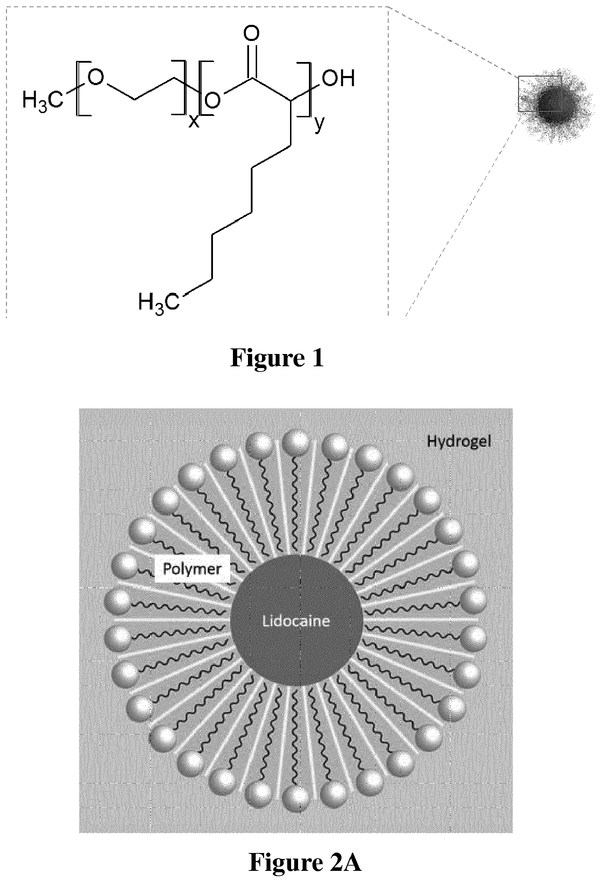

**Figure 1** represents a block copolymer mPEG-poly(caprylic acid) (5.5 kDa) used in a composition of the invention wherein x is 40-50 and y is 20-30.

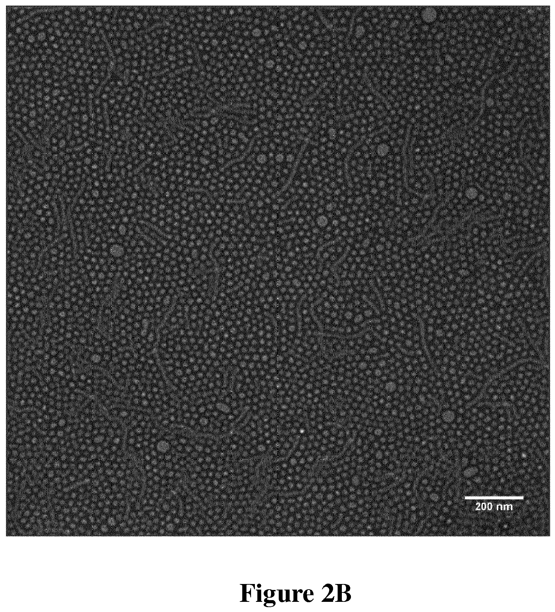

**Figure 2** represents the formed micelles of a block copolymer of methoxy poly-ethylene glycol (mPEG) and poly(caprylic acid) forming a lipophilic core for loading the lidocaine. A: Schematic representation of the formed micelles wherein the mPEG hydrophilic domains of the block copolymer form the outside of the micelles and the caprylic acid chains form the hydrophobic core of the micelles, where the amide or ester local anesthetics are loaded. **B:** electron microscopy image of the block copolymer of methoxy poly-ethylene glycol (mPEG) and poly(caprylic acid) micelles.

### Detailed Description of the invention

The expression "a block copolymer of methoxy poly(ethylene glycol) (mPEG) and poly(caprylic acid)" in the context of the invention refers to a copolymer described in Figure 1. Examples of suitable PEG polymers include those with molecular weights of up to 5'000, such as mPEG350, mPEG550, mPEG750, mPEG2'000 and the like. According to a preferred embodiment, mPEG molecular weight is about 2'000 Da.

The expression "amide or ester local anesthetics" refers to a local anaesthetic agent comprising **i)** an optionally substituted lipophilic aromatic ring, usually an unsaturated benzene ring; **ii)** an intermediate bond, such as a hydrocarbon connecting chain, comprising an ester (-C(O)-O-) or amide (-HNC(O)-) linkage: the intermediate bond determines the classification of local anaesthetic and **iii)** a terminal hydrophilic group such as a tertiary amine and proton acceptor. Amide local anesthetics comprise lidocaine, bupivacaine, ropivacaine, levobupivacaine, mepivacaine, etidocaine or a pharmaceutically acceptable salt thereof and combination thereof. Ester local anesthetics comprise tetracaine, procaine, chloroprocaine, dibucain, dyclonin, cocaine, benzocaine, or a pharmaceutically acceptable salt thereof and combination thereof.

The term "buffering agent" refers to an agent allowing to provide and maintain pH of a solution between 8-10 and is exemplified by, but not limited to sodium phosphate monobasic dihydrate, sodium phosphate dibasic dihydrate, potassium phosphate monobasic, potassium phosphate dibasic, sodium carbonate and sodium bicarbonate.

The expression "gelling agent" refers to an agent allowing to prepare a gel form of the composition of the invention and is exemplified by, but not limited to crosslinked polyacrylic acid polymers such as carbomers, for example Carbopol® Ultrez 10, hyaluronic acid and the salts thereof such as sodium hyaluronate, cellulose such as hydroxypropylcellulose, hydroxypropylmethylcellulose, methylcellulose, sodium carboxymethyl cellulose, alginic acid and alginate salts thereof such as sodium alginate, guar gum, xantham gum and carrageenan.

According to a particular aspect, the block copolymers self-assembly in aqueous medium to form spherical micelles with a core formed by the hydrophobic caprylic acid polymer segments and a surrounding shell consisting of the PEG polymer. The amide or ester local anesthetic of the invention is loaded into a micelle by a method as described herein and the resulting micelles have an average diameter of 20 to 100 nm, preferably 50 to 60 nm.

The active agent can also be adsorbed to the micelle or present in solution at low concentration.

The term "efficacy" of a treatment according to the invention can be measured based on the decreased level of experiencing of pain by a subject. Pain assessment and measurement is a multidimensional observational assessment of patients' experience of pain and include evaluation of factors such as pain intensity, location, duration and description. A patient's self-report is the most used measure of pain and commonly the patients' asses the pain on the numerical scale 0-10 (with 0 being no pain at all, and 10 the worst pain). For example, the efficacy of a treatment according to the invention can be measured by its impact on a level of experiencing pain. A response is achieved when the subject experiences partial or total reduction of a level of experiencing pain.

The term "efficacy" of a treatment of a premature ejaculation according to the invention can be measured based on the increased time before a subject expels semen after start of a sexual activity. A patient's self-report is the most used measure of efficacy of a treatment. A response is achieved when the subject experiences increased time before expelling semen after start of a sexual activity.

As used herein, "treatment" and "treating" and the like generally mean obtaining a desired pharmacological and physiological effect, in particular reducing the sensation of topical pain, irritation, inflammation or rashes. The effect may be prophylactic in terms of preventing or partially preventing pain and/or may be therapeutic in terms of a partial or complete reduction of level of experiencing pain attributed to a disease and/or a condition.

In the context of the invention, "treatment" and "treating" of a premature ejaculation mean obtaining a desired pharmacological and physiological effect. The effect may be prophylactic in terms of preventing or partially preventing of a premature ejaculation and/or may be therapeutic in terms of a partial or complete cure of a premature ejaculation.

The term "subject" as used herein refers to mammals. For examples, mammals contemplated by the present invention include human, primates, domesticated animals such as cattle, sheep, pigs, horses, laboratory rodents and the like.

The term "pharmaceutical composition" refers to preparations which are in such a form as to permit biological activity of the active ingredient(s) to be unequivocally effective and which contain no additional component which would be toxic to subjects to which the said composition would be administered.

Pharmaceutically acceptable salts are those derived from such organic and inorganic acids such as: acetic, lactic, citric, cinnamic, tartaric, succinic, fumaric, maleic, malonic, mandelic, malic, oxalic, propionic, hydrochloric, hydrobromic, phosphoric, nitric, sulfuric, glycolic, pyruvic, methanesulfonic, ethanesulfonic, toluenesulfonic, benzoic, palmoic and similarly known acceptable acids.

### Composition of the invention

According to one aspect, is provided a topical pharmaceutical composition comprising a block copolymer of methoxy poly(ethylene glycol) (mPEG) and poly(caprylic acid) and at least one amide or ester local anesthetics.

According to a particular aspect, the said at least one amide or ester local anesthetics is partly loaded in the interior of the hydrophobic core of the micelles formed by the said block copolymer, the rest remaining in solution. The loading in the core of the micelles is achieved through hydrophobic interactions of the hydrophobic domain of the block copolymer with the anesthetic molecules as schematized on Figures 2A.

According to another particular aspect, topical compositions of the invention comprise at least one amide local anesthetics.

According to a further particular aspect, topical compositions of the invention comprise lidocaine.

According to another particular aspect, topical compositions of the invention comprise at least one ester local anesthetics.

According to another aspect, topical pharmaceutical compositions of the invention further comprise at least one further pharmaceutically acceptable carrier, diluent or excipient thereof.

According to another further aspect, topical pharmaceutical compositions further comprise at least one gelling agent.

According to another further aspect, topical pharmaceutical compositions of the invention further comprise at least one solubilizing agent.

According to another further aspect, topical pharmaceutical compositions of the invention further comprise at least one non-active agent such as a conserving agent, a colorant and/or a perfume.

According to a particular aspect, compositions of the invention are analgesic or pain relief compositions, in particular for application to abrasions, rashes, eruptions or cuts in the skin or irritations of the eye.

According to a particular embodiment, compositions according to the invention are for topical administration, in particular on skin, mucosa or in the eye.

According to a further particular aspect, compositions of the invention, the invention provides an aqueous topical composition, wherein the topical amide or ester anesthetic is present in an amount of 2 mg per ml to 20 mg per ml of the composition. Typically, the aqueous topical composition comprises an anesthetic in an amount of 10 mg per ml to 20 mg per ml of the composition, preferably, in an amount of 15 mg per ml to 20 mg per ml of the composition, and more preferably, in an amount of about 16 mg per ml of the composition.

According to a further particular aspect, compositions of the invention, the invention provides an aqueous gel composition, wherein the topical amide or ester anesthetic is present in an amount of 2 mg per ml to about 16 mg per ml of the composition, and is suitable for topical administration to the eye.

According to a particular aspect, compositions of the invention are oral or buccal compositions.

According to a particular aspect, compositions of the invention are ophthalmic compositions, in particular as eye drop composition.

According to a particular aspect, compositions of the invention are penile desensitizing composition.

According to a particular aspect, compositions of the invention are vaginal compositions. Compositions for topical admiration may be in the form of suspensions, solutions, gels, hydrogels, lotions or emulsions, cream, foam, patch, sublingual droplets, buccal patches or films, buccal sprays, vaginal tablet and may further contain composition agents including, but not limited to, suspending, stabilizing, and dispersing agents.

In another particular aspect, compositions according to the invention are adapted for delivery by single or multiple administrations.

According to a particular embodiment, compositions of the invention are veterinary compositions.

Further materials as well as composition processing techniques and the like are set out in Part 5 of Remington's "The Science and Practice of Pharmacy", 22nd Edition, 2012, University of the Sciences in Philadelphia, Lippincott Williams & Wilkins*,* which is incorporated herein by reference.

### Methods of preparing a composition of the invention

According to another aspect, is provided a process for the preparation of a topical anesthetic pharmaceutical composition, said process comprising the following steps:
a) Dissolving a block copolymer mPEG-poly(caprylic acid) in an organic solvent such as acetone or ethanol, typically at a concentration of 500-600 mg per mL;
b) Adding at least one amide or ester local anesthetics to the solution, typically at a concentration of 150-200 mg per mL;
c) Adding the obtained mixture to an aqueous buffered solution at a pH ≥ pKa + 0.5 - 2.5 (e.g. for lidocaine, at a pH from about 8-10);
d) Homogenizing the obtained mixture by sonication or high-pressure homogenization;
e) Removing the organic solvent by evaporation or diafiltration;
f) Obtaining a colloidal micellar solution comprising at least one amide or ester local anesthetics.

According to a particular aspect, is provided a process for the preparation of a topical anesthetic pharmaceutical composition according to the invention, wherein a solubilizing agent (e.g. such as mPEG-poly(caprylic acid)) is added under step a).

According to a particular further aspect, is provided a process for the preparation of a topical anesthetic pharmaceutical composition according to the invention wherein the homogenization under step d) is carried out by sonication (e.g. 20 % amplitude for 20 to 60 min, on ice).

According to another particular further aspect, is provided a process for the preparation of a topical anesthetic pharmaceutical composition according to the invention wherein the homogenization under step d) is carried out by high-pressure homogenization (e.g. under 200 bars for about 20 to 60 min).

According to another further aspect, a process of the invention may further comprise a step after step e) of adding a gelling agent such as Carbopol for obtaining a composition according to the invention in the form of a colloidal gel under step f).

### Mode of administration

Compositions of this invention may be administered or delivered in any manner including, but not limited to, orally, sublingually, bucally, dermally, topically on skin or mucosa, via intrarectal, intravaginal, intranasal or intraocular administration, or combinations thereof.

In another particular embodiment, compositions according to the invention are administered by topical administration on the skin, mucosa or in the eye.

According to a particular embodiment, compositions according to the invention can be impregnated into or coated on the surface of a wound dressing, occlusive bandage, sponge, gauze or condom.

According to a particular embodiment, composition of the invention can be incorporated in a bandage composition suitable for application from aerosol containers such as spray-spun bandages to provide an adherent protective film bandage on the afflicted area.

The dosage administered, as single or multiple doses, to an individual will vary depending upon a variety of factors, including pharmacokinetic properties, subject conditions and characteristics (sex, age, body weight, health, and size), extent of symptoms, concurrent treatments, frequency of treatment and the effect desired.

### Combination

According to one embodiment of the invention, the compositions according to the invention can be administered alone or in combination with a co-agent.

According to one aspect, compositions of the invention are to be administered in combination with at least one further therapeutic molecule useful in the prevention and/or treatment of pain.

According to one aspect, compositions of the invention are to be administered in combination with at least one further therapeutic molecule useful in the prevention and/or treatment of a premature ejaculation.

According to another aspect, compositions of the invention can be administered simultaneously, optionally in the same composition, in combination with at least further one therapeutic molecule useful for the prevention and/or treatment of pain or a premature ejaculation.

The invention encompasses the administration of a composition according to the invention, wherein the said composition is administered to an individual prior to, simultaneously or sequentially with other therapeutic regimens or co-agents (e.g. multiple drug regimens), in a therapeutically effective amount. Compositions according to the invention that are administered simultaneously with said co-agents can be administered in the same or different composition(s) and by the same or different route(s) of administration. For example, the compositions of the invention can be mixed with other micellar formulations or dissolved in the outer phase of said other micellar formulations.

### Use according to the invention

The compositions of the invention are useful for local dermal, mucosal or ocular anaesthesia. According to another aspect, the compositions of the invention are useful to prevent pain associated with any type of perforation of skin by a needle, intravenous catheter insertion, blood sampling, superficial or minor surgical procedures such as in advance of injected local anaesthetics for minor surgery and biopsies, skin cleansing or debridement for example in skin ulcers, laser treatments such as laser hair or tattoo removal, tattooing.
According to another aspect, the compositions of the invention are useful to treat pain associated with insect bites, skin eruptions, minor skin trauma such as scrapes, exposure to sun (sunburn), plant-induced urticarial rash i.e., hives, painful or pruriginous skin eruptions such as associated with chicken pox and zona (also known shingles or herpes zoster), contact dermatitis for example from exposure to allergens (allergic contact dermatitis) or irritants

(irritant contact dermatitis), skin diseases such as eczema or acne, skin irritation such as caused by abrasives impregnated in clothing rubbing the skin, skin diseases such as eczema or acne.
According to another aspect, the compositions of the invention are useful to prevent pain associated with mucosal diseases such as mouth ulcers and STD.
According to one aspect, the compositions of the invention are useful to prevent pain associated with eye irritation such as allergic, viral or bacterial conjunctivitis and ocular trauma (blunt trauma, blowout fracture of the orbital walls, lid laceration, intraocular foreign body injury, corneal abrasion, retinal/vitreous injury).
According to one aspect, the compositions of the invention are useful to prevent and/or treat premature ejaculation.

According to an aspect, the compositions are contemplated for use on topical or superficial procedures carried out on various tissues and organs, e.g., in dermatology, dentistry, ear, nose, and throat (ENT), urology, and gynecology

### Patients

In an embodiment, subjects according to the invention are at risk of or experiencing topical pain associated with topical inflammation, abrasions, rashes, eruptions, cuts or irritations or associated with skin perforation before superficial or minor surgical or injection procedures. In an embodiment, subjects according to the invention are at risk of or experiencing premature ejaculation.

The present invention is not to be limited in scope by the specific embodiments described herein, which are intended as single illustrations of individual aspects of the invention. Indeed, various modifications of the invention, in addition to those shown and described herein will become apparent to those skilled in the art from the foregoing description. The invention having been described, the following examples are presented by way of illustration, and not limitation.

### EXAMPLES

The following abbreviations refer respectively to the definitions below:
**mPEG** (methoxy poly(ethylene glycol)).

### Example 1: Preparation of a composition according to the invention

A composition of the invention was prepared by a process comprising the following steps:
**Step 1)** 1.1 g of mPEG-poly(caprylic acid) **(****Figure 1****)** was dissolved in 2 mL acetone at ambient temperature. Sonication bath was used for about 5 minutes to accelerate the dissolution of the polymer.
**Step 2)** 350 mg of lidocaine were added to the solution as an amide anaesthetic active agent;
**Step 3)** The obtained mixture was added dropwise to water containing 10 mM bicarbonate buffer pH 10;
**Step 4)** The obtained mixture was then homogenized by sonication (e.g. 20 % amplitude, on ice to prevent heating of the formulation, for 40 min);
**Step 5)** Acetone was removed by evaporation under lowered pressure (mixture heated at 60 °C; decreasing pressure from 350 to 180 mbar over 20 min, 180 mbar for 90 min) until the leftover impurities of acetone were measured to be less than 250 ppm [check by paper indicator or Gaz Chromatography for acetone quantification (according to European Pharmacopoeioa, 9th Edition)]. Alternatively, diafiltration could be used to remove acetone
**Step 6)** A colloidal micellar solution was obtained having a concentration of lidocaine ranging from 12 to 18 % w./v., a micelle size (Z-average) comprised between 50 and 60 nm and a polydispersity index (PDI) between 0.1 and 0.15 as measured by dynamic light scattering.

In some preparations of a topical composition of the invention in a gel form, a gelling agent is further added to a solution obtained after step 5) to obtain a gel having a concentration of lidocaine ranging from 1.3% to 1.7% m/v.

### Example 2: Characterization of a composition according to the invention

The topical liquid composition (without a gelling agent) obtained from a process of the invention as described in Example 1 in steps 1-6 has been characterized for its stability over time and in different temperature of storage as follows: The formulations prepared at 1.8, 9 and 16 mg/mL in 10 mM buffer were stored in glass vials (1-2 mL per vial) at 2 to 8, 25 and 40 °C. At each time-point, one vial at each temperature was analysed. Characterization of the formulation was made by the visual aspect, lidocaine concentration, pH and particle size.

Those tests indicated that the formulation was stable at 5 °C, 25 °C and 40 °C for at least 2 months as assessed by visual examination, HPLC (lidocaine quantification) and particle size measurements (zetasizer™, Malvern Instruments). A decrease of pH (from 10 to 9 at 1 month, 10 to 8 at 2 months) was observed at 40 °C. The pH was stable for 2 months at 5 °C and decreased by 1 unit at 25 °C after 2 months.

Therefore, a liquid composition according to the invention is stable for at least 2 months, except for pH, which supports that this composition is suitable for pharmaceutical production if higher buffer strength (50 mM) is used.

### Example 3: Efficacy of a composition according to the invention

A viscosified composition obtained from a process of the invention with the addition of a gelling agent at the end of step 5), as described in Example 1 has been tested for its efficacy as compared to EMLA® (a mixture of 2.5 % lidocaine and 2.5% prilocaine).

A clinical study was performed on 5 volunteers as follows:

### Equipment:

- Formulations tested:
   - EMLA® (Aspen Pharma Schweiz GmbH, Baar, Switzerland)
   - Topicaine (gel, OTC in the USA) from ESBA Laboratories Inc., USA
   - Formulation of the invention with Lidocaine obtained under Example 1, provided in syringes (1 mL) numbered (double-blind, except for EMLA® (because of the different appearance))
- Roller and needle length: 0.75 mm
- Treatment zone delimiter ("punched" electrode plasters: identical surface (∼ 1.5 cm in diameter) for all treatments)
- Occlusive dressing
- Lancets
- Disinfectant (70% alcohol)
- Follow-up sheet (level of pain (0 - 10), observations, one per volunteer)
- Heat applicator (ColdHot™ Pack, 3M).

### Application protocol

The following application protocol is followed:
1) Delimitation of treatment areas
2) Disinfection
3) Roller application for the arm concerned: the roller was pressed against the skin and rolled over 4 cm 3x up and down then 3x side to side as if drawing a cross
4) Application of formulations under occlusion: the formulations were applied in an adhesive 1 mm high sticker with a 1 cm diameter hole in the middle to prevent the formulation to spread on the skin when occluded. The formulation contained in the hole was then covered by an occlusive adhesive plastic from 3M (Tegaderm™)
5) Heat application for the arm concerned
6) 15 minutes after application: needle test: a 30 G needle was inserted 2 mm deep into the skin
7) 30 minutes after application: needle test
8) 45 minutes after application: needle test.

The formulations are topically applied on the external part of the arm on 18 zones per volunteer (9 on each arm, corresponding to the three formulations above with three durations of application). The applications are conducted on both arms:
- one arm with roller + heat applicator (throughout the duration of the study);
- the other arm without.

The applications with the roller are conducted with crossings: 3 times vertically, 3 times horizontally.
The volume of the formulations that is applied is ∼300 µL/zone (corresponding to ∼ 2 mL per formulation per volunteer)

A follow-up sheet by volunteer is completed: Pain level (0 - 10) for each needle test and observations throughout the study. Pain level: 0 corresponds to no sensation (complete anesthesia); 10 corresponds to sting without anesthesia.

The formula of the invention provided a similar efficacy profile as compared to EMLA® as measured by pain evaluation.

Therefore, a composition of the invention can provide similar anaesthetics effect as compared to known formulation of lidocaine, despite the fact that it contains less lidocaine (1.6% *vs*. 2.5% in EMLA®) and has the advantage of not containing prilocaine.

## Claims

1. A topical pharmaceutical composition comprising a block copolymer of methoxy poly-ethylene glycol (mPEG) and poly(caprylic acid) and at least one amide or ester local anesthetics.

2. A topical pharmaceutical composition according to claim 1 wherein the said at least one amide or ester local anesthetics is loaded in the interior of the micelle formed by the said block copolymer.

3. A topical pharmaceutical composition according to claim 1 or 2, wherein the said at least one amide anesthetics is lidocaine.

4. A topical pharmaceutical composition according to any one of claims 1 to 3, further comprising at least one further pharmaceutically acceptable carrier, diluent or excipient thereof.

5. A topical pharmaceutical composition according to any one of claims 1 to 4, further comprising at least one gelling agent.

6. A topical pharmaceutical composition according to any one of claims 1 to 5, further comprising at least one solubilizing agent.

7. A topical pharmaceutical composition according to any one of claims 1 to 6, wherein the topical amide or ester anesthetic is present in an amount of 2 mg per ml to 20 mg per ml of the composition.

8. A topical pharmaceutical composition according to any one of claims 1 to 8, wherein said composition is an oral or buccal composition.

9. A topical pharmaceutical composition according to any one of claims 1 to 8, wherein said composition is ophthalmic composition.

10. A topical pharmaceutical composition according to any one of claims 1 to 8, wherein said composition is a mucosal composition.

11. A topical pharmaceutical composition according to any one of claims 1 to 8, wherein said composition is a penile desensitizing composition.

12. A topical pharmaceutical composition according to any one of claims 1 to 11 for use for local anaesthesia or for the prevention and/or treatment of topical inflammation, abrasions, rashes, eruptions, cuts or irritations or premature ejaculation.

13. A process for the preparation of a topical anesthetic pharmaceutical composition, said process comprising the following steps:
a) Dissolving a block copolymer mPEG-poly(caprylic acid) in an organic solvent such as acetone or ethanol;
b) Adding at least one amide or ester local anesthetics to the solution, typically at a concentration of 150-200 mg per mL;
c) Adding the obtained mixture to an aqueous buffered solution at a pH ≥ pKa + 0.5 - 2.5;
d) Homogenizing the obtained mixture by sonication or high-pressure homogenization;
e) Removing the organic solvent by evaporation or diafiltration;
f) Obtaining a colloidal micellar solution comprising at least one amide or ester local anesthetics.

14. A process according to claim 13, further comprising a further step after step e) of adding a gelling agent such as Carbopol.

15. A topical article for anaesthesia such as wound dressing or bandage, sponge, gauze or condom coated or impregnated with a composition according to any one of claims 1 to 11 or obtainable from a process according to claims 13 or 14.
